**Europäisches Patentamt**

**European Patent Office**

**Office européen des brevets**

(19)

(11) Publication number: **0 148 565**
**B1**

(12) **EUROPEAN PATENT SPECIFICATION**

(45) Date of publication of patent specification: **03.08.88**

(51) Int. Cl.⁴: **A 61 G 9/00**

(21) Application number: **84307479.0**

(22) Date of filing: **30.10.84**

(54) **Urine collection device.**

(30) Priority: **04.11.83 GB 8329552**

(43) Date of publication of application:
**17.07.85 Bulletin 85/29**

(45) Publication of the grant of the patent:
**03.08.88 Bulletin 88/31**

(84) Designated Contracting States:
**BE DE FR NL SE**

(56) References cited:
**FR-A-1 061 419**
**US-A-3 473 172**
**US-A-3 963 020**
**US-A-4 023 216**

(73) Proprietor: **Rocket of London Limited**
**Imperial Way**
**Watford Hertfordshire (GB)**

(72) Inventor: **Edwards, Roy Desmond**
**c/o Beehive Mouldings Ltd Imperial Way**
**Watford Hertfordshire (GB)**

(74) Representative: **Lynd, Michael Arthur et al**
**MARKS & CLERK 57 Lincoln's Inn Fields**
**London WC2A 3LS (GB)**

Courier Press, Leamington Spa, England.

## Description

This invention relates to a device for collecting urine and delivering it to a specimen bottle, and to the combination of a said device and a said specimen bottle.

Specimens of urine are frequently required from patients to establish the presence or absence of substances affecting the health of the patients. A small cylindrical container or bottle of standard size is generally employed to hold the specimen. Conventionally the filling of such a container with such a specimen has been somewhat haphazard and unhygienic and various proposals have been made for improving the operation.

In one such proposal a disposable paper funnel is provided at its lower, narrower end with an adhesive label whereby it can be affixed to the outer rim of a said standard or 'universal' container. In a second proposal a disposable paper funnel is affixed to a screw cap having an axial passage therethrough which cap may be screwed on to the container. After the container has been fitted the screw cap may be removed and a conventional screw cap used to seal the container. Both of these prior art proposals still present problems since it is not easy for the user to gauge when the container is full nor to dispose of any surplus urine in the funnel without at the same time emptying the container.

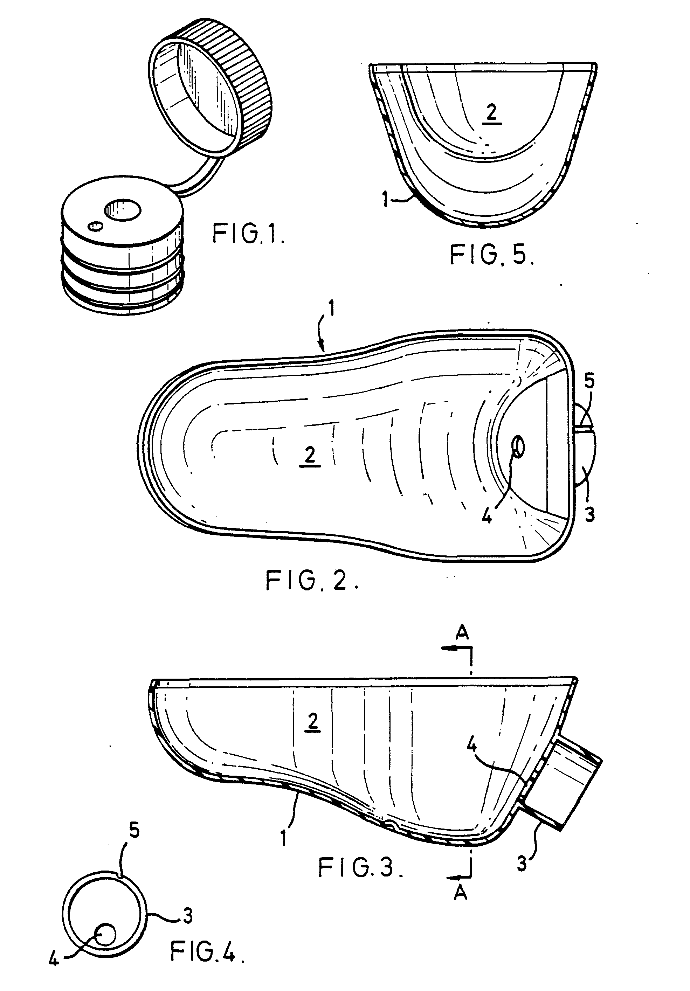

Yet another proposal provides a test tube having a plastics plug. The plug has a lid therefor connected to it be a flexible integral web and is provided with a central aperture for receiving urine and a bleed tube. An embodiment of this proposal is shown diagrammatically in Fig. 1 of the accompanying drawings. Whilst this proposal constitutes an at least partial solution it is much more expensive and complex to manufacture.

US Patent Specification No. 4023216 discloses a trough for collecting the 'poorly defined urinary efflux of a urinating human female in a normal standing position', which trough is provided at its forward end with a forwardly and downwardly inclined discharge conduit. The disclosure of this US Patent Specification is not concerned with solving the problem of filling specimen bottles in a satisfactory manner.

US Patent Specification No. 3473172 discloses a 'female urinal' comprising a funnel-shaped member adapted to be secured to an enclosed bedpan by means of a bayonet fitting. The invention is not concerned with the satisfactory filling of urine specimen bottles.

According to the present invention there is provided a device for collecting a specimen of urine, device comprises a hollow container portion and, integrally formed therewith a plug portion adapted to fit into the neck of a so-called 'universal' urine specimen bottle, said plug portion having an aperture or bore therethrough through which urine may pass from the container portion into a said bottle, and characterised in that said plug portion is provided with a means for permitting escape of air from a said specimen bottle as it fills with urine, said second aperture or conduit being so dimensioned that, in use, surface tension effects will block said means and prevent any substantial leakage of urine therethrough when the specimen bottle is full, the diameter of said aperture or bore being so selected that with said means blocked by urine, urine within a said specimen bottle is prevented from leaking through said first aperture or bore when the device with specimen bottle are inverted.

Although the hollow container portion of the device may be of any suitable shape, e.g. frustoconical, it is preferred that it be shaped to have a generally anatomical fit to the human body. The device is preferably sterilizable, e.g. by heat or by irradiation and may be made to be disposable after use or to be reusable.

The means preferably constitutes an axially extending groove in said plug portion. The groove is preferably located about the periphery of the plug portion at such a position that it is blocked by urine before a said specimen bottle is filled with urine.

The device is of such a composition and the aperture or bore through the plug portion preferably of such a diameter that the volume of urine which can be contained in said specimen bottle is so light in weight that a said volume can be retained within a said specimen bottle even when it is upside down, so that excess urine in the container portion may be disposed of.

Suitable materials are transparent or translucent materials, e.g. glass, or plastics, such as polyethylene or polypropylene.

An embodiment of the device of the present invention will now be described by way of reference to Figs 2 to 5 of the accompanying drawings, in which:

Fig. 2 is a plan view of a said device;

Fig. 3 is a cross-section side elevation of the device of Fig. 2;

Fig. 4 is a section through the plug portion 20 of the device of Figs. 2 and 3 and

Fig. 5 is a section along the line A—A of Fig. 3.

Referring to Figs. 2 to 5 of the drawings a polyethylene urine collection device comprises a hollow container portion adapted to encompass the uro-genital area of the human female anatomy and taking the form of a hemi-pear-shaped trough.

A plug portion is integrally moulded onto the exterior of the container portion adjacent a deepest point of the container portion and communicates with the interior of the container portion *via* an aperture. The external diameter of the plug portion is selected to render the plug portion a push fit into a specimen bottle which may conveniently be a so-called 'standard' or 'universal' specimen bottle and may be, for example, 21.9 mm. The diameter of the aperture is so selected in conjunction with the specimen bottle with which the device is to be employed as to impede any flow of urine from the bottle through the aperture. A suitable diameter is 4.7 mm.

An axial groove for allowing air to escape from a said specimen bottle is formed in the outer cylindrical wall of plug portion 3 of 1.5 mm radius. This radius is such that in use surface tension effects will cause the groove to become sealed by urine as the urine reaches the lower rim of the plug portion. The groove is located in the lower part of the cylindrical wall so that in use urine in the specimen bottle will contact the bottom of the groove and seal it to prevent further egress of air from the specimen bottle thus preventing further filling of the specimen bottle.

Excess urine can be emptied from the container portion without urine in the specimen bottle leaving the bottle through the aperture (because of the selected diameter of the aperture), whereafter the collection device can be unplugged from the bottle and replaced by a conventional bottle cap or plug.

The device of the present invention can provide a hygienic and convenient way of filling a specimen bottle with urine without spillage or overfilling.

## Claims

1. A device for collecting a specimen of urine, which device (1) comprises a hollow container portion (2) and, integrally formed therewith a plug portion (3) adapted to fit into the neck of a so-called 'universal' urine specimen bottle, said plug portion (3) having an aperture or bore (4) therethrough through which urine may pass from the container portion (2) into said bottle, and characterised in that said plug portion (3) is provided with a means (5) for permitting escape of air from a said specimen bottle as it fills with urine, said means (5) being so dimensioned that, in use, surface tension effects will block said means (5) with urine and prevent any substantial leakage of urine therethrough when the specimen bottle is full, the diameter of said aperture or bore (4) being so selected that, with said means (5) blocked by urine, urine within a said specimen bottle is prevented from leaking through said aperture or bore (4) when the device with specimen bottle are inverted.

2. A device according to Claim 1, characterised in that said hollow container portion (2) is generally frustoconical in shape.

3. A device according to Claim 1, characterised in that hollow container portion (2) is adapted to encompass and fit the uro-genital area of the human female anatomy.

4. A device according to Claim 3, characterised in that the hollow container portion (2) is in the form of a hemi-pear-shaped trough.

5. A device according to any preceding claim, characterised in that the device (1) is formed of a sterilizable material.

6. A device according to Claim 5, characterised in that the device (1) is formed of glass or of plastics material.

7. A device according to Claim 6, characterised in that the device (1) is formed of polyethylene or polypropylene.

8. A device according to any preceding claim, wherein said means (5) comprises a generally axially extending groove (5) in said plug portion (3).

9. A device according to Claim 8, characterised in that said groove (5) is located on an outer face of said plug portion (3).

## Patentansprüche

1. Vorrichtung zum Sammeln einer Urinprobe, die (1) einen ausgehöhlten Behälterteil (2) und einteilig hiermit ausgebildet einen Stopfenteil (3) derartiger Ausbildung umfat, daß er in den Hals einer sog. Urinprobenuniversalflasche paßt, wobei dieser Stopfenteil (3) eine durchgehende Öffnung oder Bohrung (4) aufweist, durch welche Urin vom Behälterteil (2) in diese Flasche treten kann, dadurch gekennzeichnet, daß dieser Stopfenteil (3) mit einer Einrichtung (5) versehen ist, die das Entweichen von Luft aus einer Probenflasche während des Vorgangs des Füllens mit Urin gestattet, wobei diese Einrichtung (5) so dimensioniert ist, daß in Benützung Oberflächenspannungseffekte diese Einrichtung (5) mit Urin blockieren und jeden wesentlichen Durchtritt von Urin hierdurch, wenn die Probenflasche voll ist, verhindert, wobei der Durchmesser dieser Öffnung oder Bohrung (4) so gewählt ist, daß bei durch Urin blockierten Einrichtungen (5) der Urin innerhalb dieser Probenflasche daran gehindert wird, durch diese Öffnung oder Bohrung (4) durchzutreten, wenn Vorrichtung mit Probenflasche umgedreht werden.

2. Vorrichtung nach Anspruch 1, dadurch gekennzeichnet, daß dieser ausgehöhlte Behälterteil (2) von im wesentlichen kegelstumpfförmiger Gestalt ist.

3. Vorrichtung nach Anspruch 1, dadurch gekennzeichnet, daß dieser ausgehöhlte Behälterteil (2) so ausgebildet ist, daß er den Urogenitalbereich der Anatomie eines weiblichen Menschem umschließt und hierzu paßt.

4. Vorrichtung nach Anspruch 3, dadurch gekennzeichnet, daß der ausgehöhlte Behälterteil (2) in Gestalt einer halbbirnenförmigen Rinne vorliegt.

5. Vorrichtung nach einem der vorhergehenden Ansprüche, dadurch gekennzeichnet, daß die Vorrichung (1) aus einem sterilisierbaren Material geformt ist.

6. Vorrichtung nach Anspruch 5, dadurch gekennzeichnet, daß die Vorrichtung (1) aus Glas oder Kunststoffmaterial geformt ist.

7. Vorrichtung nach Anspruch 6, dadurch gekennzeichnet, daß die Vorrichtung (1) aus Polyäthylen oder Polypropylen geformt bzw. gebildet ist.

8. Vorrichtung nach einem der vorhergehenden Ansprüche, dadurch gekennzeichnet, daß diese Einrichtung (5) eine im wesentlichen axial sich erstreckende Nut (5) in diesem Stopfenteil (3) umfaßt.

9. Vorrichtung nach Anspruch 8, dadurch gekennzeichnet, daß diese Nut (5) in einer Außenfläche dieses Stopfenteils (3) angeordnet ist.

**Revendications**

1. Dispositif pour collecter un échantillon d'urine, ce dispositif (1) comprenant une région creuse (2) formant récipient et, solidaire de celle-ci, une région (3) formant fiche adaptée à se fixer à l'intérieur du goulot d'une bouteille pour échantillon d'urine dite universelle, ladite région formant fiche (3) étant traversée par une ouverture ou alésage (4) qui permet le passage d'urine de la région formant récipient (2) vers ladite bouteille, et caracérisé en ce que ladite région formant fiche (3) comprend des moyens (5) pour permettre l'évacuation de l'air de ladite bouteille pour échantillon lorsqu'elle se remplit d'urine, lesdits moyens (5) étant dimensionnés de sorte que, lors de l'utilisation, des effets de tension de surface bloquent ces moyens avec de l'urine et empêchent toute fuite substantielle d'urine par leur intermédiaire lorsque la bouteille pour échantillon est pleine, le diamètre de l'ouverture ou alésage (4) étant choisi de telle sorte que, lesdits moyens (5) étant bloqués par l'urine, de l'urine contenue dans la bouteille pour échantillon, ne puisse s'échapper par cette ouverture ou alésage (4) quand le dispositif comprenant la bouteille pour échantillon est renversé.

2. Dispositif conforme à la revendication 1, caractérisé en ce que ladite région creuse (2) formant récipient est de section générale tronconique.

3. Dispositif conforme à la revendication 1, caractérisé en .ce que ladite région creuse (2) formant récipient est adaptée à envelopper tout en s'ajustant autour de la région urogénitale de l'anatomie de la femme.

4. Dispositif conforme à la revendication 3, caractérisé en ce que ladite région creuse (2) formant récipient a la forme d'une vasque conformée en demi-poire.

5. Dispositif conforme à l'une des revendications précédentes, caractérisé en ce que ce dispositif (1) est réalisé en matériel stérilisable.

6. Dispositif conforme .à la revendication 5, caractérisé en ce que ce dispositif (1) est réalisé en verre ou en matière plastique.

7. Dispositif conforme à la revendication 6, caractérisé en ce que ce dispositif (1) est réalisé en polyéthylène ou en polypropylène.

8. Dispositif conforme à l'une des revendications précédentes, caractérisé en ce que lesdits moyens (5) comprennent une rainure (5) s'étendant sensiblement axialement dans ladite région (3) formant fiche.

9. Dispositif conforme à la revendication 8, caractérisé en ce que ladite rainure (5) est située sur une face externe de ladite région (3) formant fiche.

0 148 565

FIG.1.

FIG.5.

FIG.2.

FIG.3.

FIG.4.